# FASCICULE DE BREVET EUROPEEN

(11) **EP 3 169 695 B1**
(45) Date de publication et mention de la délivrance du brevet: **13.11.2019**
(21) Numéro de dépôt: 15753722.6
(22) Date de dépôt: 16.07.2015
(51) Int. Cl.: A23J 1/00, C07K 1/14, C07K 1/34, C07K 1/36, C07K 14/405

(54) **PROCEDE D'EXTRACTION DES PROTEINES SOLUBLES DE BIOMASSES DE MICROALGUES**
VERFAHREN ZUR EXTRAKTION VON LÖSLICHEN PROTEINEN AUS MIKROALGENBIOMASSE
METHOD FOR EXTRACTING SOLUBLE PROTEINS FROM MICROALGAL BIOMASS

(30) Priorité: 18.07.2014 FR 1456944
(43) Date de publication de la demande: 24.05.2017
(73) Titulaire: Corbion Biotech, Inc., South San Francisco, CA 94080 (US)
(72) Inventeur: PATINIER, Samuel, F-59890 Quesnoy sur Deule (FR)
(74) Mandataire: Cabinet Becker et Associés
(86) Numéro de dépôt international: PCT/FR2015/051940
(87) Numéro de publication internationale: WO 2016/009145

(56) Documents cités:
- SU-A1- 731 935
- ALINA-VIOLETA URSU ET AL: "Extraction, fractionation and functional properties of proteins from the microalgae Chlorella vulgaris", BIORESOURCE TECHNOLOGY, vol. 157, 1 mars 2014 (2014-03-01), pages 134-139, XP055108068, ISSN: 0960-8524, DOI: 10.1016/j.biortech.2014.01.071
- COUSTETS M ET AL: "Flow Process for Electroextraction of Total Proteins from Microalgae", JOURNAL OF MEMBRANE BIOLOGY, SPRINGER, XX, vol. 246, no. 10, 11 avril 2013 (2013-04-11), pages 751-760, XP035326801, ISSN: 0022-2631, DOI: 10.1007/S00232-013-9542-Y [extrait le 2013-04-11]

## Description

La présente invention concerne un procédé d'extraction des protéines solubles de la biomasse de microalgues.

La présente invention concerne également les isolats de protéines de microalgues ainsi obtenus.

### Présentation de l'état de l'art

Il est bien connu de l'homme du métier que les chlorelles sont une source potentielle de nourriture, car elles sont riches en protéines et autres nutriments essentiels.

Elles sont décrites comme renfermant 45% de protéines, 20% de matières grasses, 20% de glucides, 5% de fibres et 10% de minéraux et de vitamines.

Etant donné leur abondance et leur profil en acides aminés, les protéines de microalgues sont ainsi considérées comme une source alternative aux protéines de soja ou de pois en Alimentation.

La fraction protéique peut être également valorisée comme agent fonctionnel dans les industries cosmétiques, voire pharmaceutiques.

Cependant, les développements en applications alimentaires des protéines de microalgues n'ont pas été significatifs, car la présence, dans lesdites fractions, de composés indésirables (telle la chlorophylle) conduit à des changements non souhaités de couleur, goût et structure des compositions alimentaires en contenant.

Pour augmenter leur potentialité en applications alimentaires et pour augmenter également leur valeur commerciale, ces protéines doivent donc être extraites des microalgues sans impacter leur structure moléculaire.

Les techniques « douces » d'extraction seraient donc nécessaires pour isoler les protéines de grandes solubilités et de bonnes propriétés technico-fonctionnelles, mais la rigidité des parois des microalgues, notamment des microalgues vertes, s'y oppose fondamentalement car elle perturbe l'extraction et l'intégrité des protéines intracellulaires.

C'est ainsi qu'au contraire des conditions physiques ou chimiques classiquement « dures » sont mises en œuvre pour rompre la paroi des microalgues.

De nombreuses études proposent ainsi des technologies de type dissolution alcaline, extraction par solvants organiques, ou homogénéisation haute pression.

Dans ces choix technologiques, la dénaturation des protéines n'était cependant pas considérée comme gênante, puisque la plupart de ces méthodes étaient développées pour des finalités d'analyses ou destinées à fournir un substrat pour la digestion enzymatique productrice d'hydrolysats protéiques.

Or, un procédé de désintégration efficace préservant l'intégrité des composants cellulaires se doit de maximaliser non seulement le rendement mais aussi la qualité des produits extraits.

En d'autres termes, un procédé de désintégration optimisé de la paroi doit par exemple éviter :
- la contamination chimique des produits ciblés,
- la mise en œuvre d'énergie de rupture trop importante ; celle-ci pouvant occasionner une dégradation ou dénaturation irréversible des molécules intracellulaires d'intérêt.

Par ailleurs, pour des productions à grande échelle, il est important que le procédé choisi soit transposable à cette échelle.

Enfin, l'introduction de cette étape de désintégration cellulaire doit être aisée et ne doit pas avoir un impact négatif sur les étapes de procédé/traitement subséquentes.

Toutes ces limitations influencent l'efficacité du procédé de désintégration et par là-même sa consommation énergétique.

C'est la raison pour laquelle la technologie de broyage à billes est préférée, car elle est considérée comme efficace pour libérer les protéines intracellulaires sous leur forme native.

Dans un broyeur à billes, les cellules sont agitées en suspension avec de petites particules sphériques. Le cassage des cellules est provoqué par les forces de cisaillement, le broyage entre les billes, et les collisions avec des billes.

La description d'un broyeur à billes approprié est par exemple faite dans le brevet US 5.330.913. Ces billes cassent les cellules pour en libérer le contenu cellulaire. On obtient alors une suspension de particules de plus petite taille que les cellules d'origine sous la forme d'une émulsion « huile dans eau ».

Cette émulsion est généralement atomisée et l'eau est éliminée, laissant une poudre sèche contenant cependant un mélange hétérogène composé de débris cellulaires, de composés solubles interstitiels et d'huile.

La difficulté à résoudre dans l'emploi de ces technologies de désintégration cellulaire est l'isolement du seul contenu intracellulaire (à l'exclusion des débris membranaires, des sucres, des fibres et des matières grasses) et la préservation, notamment, de la qualité de la charge protéique.

Dans le cas de la microalgue du genre *Tetraselmis sp,* Anja Schwenzfeier et al (Bioresource Technology, 2011, 102, 9121 - 9127) ont proposé un procédé garantissant la solubilité et la qualité de l'aminogramme des protéines isolées et débarrassées des contaminants (telles les substances colorantes) comprenant les étapes suivantes :
- désintégration cellulaire par broyage à billes,
- centrifugation de la suspension de microalgues broyées,
- dialyse du surnageant,
- passage sur résine échangeuse d'ions,
- dialyse de l'éluat,
- décoloration, puis
- lavage et remise en solution.

Cependant, ce procédé de laboratoire (pour traiter 24 g de biomasse) n'est pas transposable à l'échelle industrielle, où le procédé de broyage à billes est plutôt mis en œuvre pour récupérer une biomasse complète.

Des solutions alternatives ont été proposées en changeant complètement la technologie de libération du contenu intracellulaire des microalgues, comme le traitement électrique à champ pulsé.

L'exposition des cellules biologiques à un champ électrique pulsé de haute intensité peut en effet altérer la structure de la membrane cellulaire.

Le champ externe provoque le chargement de la membrane. A une tension transmembranaire suffisante (0,5-1 V), l'arrangement moléculaire des phospholipides change, ce qui conduit à faire perdre à la membrane son rôle de barrière, la rendant perméable. En fonction des conditions mises en œuvre, cette perméabilisation membranaire peut être réversible ou irréversible.

Pour une extraction efficace des composés intracellulaires, il reste cependant conseillé à l'homme du métier utilisateur de cette technologie de provoquer une perméabilisation irréversible de la membrane, ce qui conduit à sa désintégration.

Cette rupture de la membrane facilite alors la libération du contenu cellulaire et, en cas d'utilisation d'une technique complémentaire d'extraction par solvant, facilite également la pénétration du solvant dans la cellule.

Cette technique, quoique prometteuse, n'est malheureusement pas extrapolable à une échelle industrielle pour traiter une biomasse produite en réacteur de 1 à 200 m³.

Il résulte qu'il demeure un besoin non satisfait de disposer d'une technologie de fragilisation des parois des microalgues apte à libérer le contenu intracellulaire sans désintégrer la cellule ni altérer la qualité de ses composants. SU731935 décrit le traitement de la biomasse de Chlorella à une température comprise entre 85 et 98°C, pendant une durée de 3-5 minutes, suivi de l'élimination de la biomasse ainsi perméabilisée par la centrifugation à 6000 rpm, pour obtenir un isolat protéique.

La société Demanderesse a trouvé que ce besoin pouvait être satisfait en combinant un procédé de perméabilisation thermique des cellules de microalgues avec des étapes de centrifugation et de séparation membranaire (microfiltration, ultrafiltration).

La société Demanderesse va ainsi à l'encontre d'un préjugé technique qui veut que les méthodes thermiques de disruption cellulaire, tout comme les forces de cisaillement provoquées par la désintégration mécanique, soient des technologies plutôt mises en œuvre pour dégrader ou dénaturer les produits issus des microalgues (Richmond, 1986, Handbook of Microalgal Mass Culture. CRC Press, Inc - Molina Grima et al., 2003, Recovery of microalgal biomass and metabolites: process options and economics, Biotechnol. Adv. 20:491-515).

Par ailleurs, une fois libérées du compartiment intracellulaire, la récupération des molécules est réalisée aisément par centrifugation et séparation membranaire, étant donné que le traitement thermique développé par la société Demanderesse ne conduit pas à la désintégration de la paroi cellulaire.

La présente invention est relative à un procédé de perméabilisation thermique de la biomasse de microalgues du genre *Chlorella* de manière à en récupérer les fractions solubles enrichies notamment en peptides et polypeptides et en oligosaccharides.

Plus précisément, le procédé selon l'invention est un procédé de préparation d'un isolat protéique de la biomasse de microalgues du genre *Chlorella,* comprenant les étapes suivantes :
- fourniture d'une biomasse de microalgues produite par fermentation,
- de manière optionnelle, lavage de la biomasse de manière à éliminer les composés solubles interstitiels,
- perméabilisation thermique de la biomasse à une température comprise entre 50 et 150 °C, de préférence 100 et 150 °C, pendant une durée comprise entre 10 secondes et 5 minutes, de préférence pendant une durée comprise entre 10 secondes et 1 minute,
- élimination de la biomasse ainsi perméabilisée par une technique de séparation solide-liquide choisie dans le groupe constitué de la filtration frontale ou tangentielle, la floculation et la centrifugation, plus particulièrement la centrifugation multi-étagée, pour obtenir une fraction soluble,
- de manière optionnelle, récupération et clarification de la fraction soluble ainsi obtenue par microfiltration de manière à la débarrasser des insolubles résiduels,
- ultrafiltration de la fraction soluble (clarifiée ou non clarifiée, selon que l'étape précédente de récupération et clarification soit respectivement effectuée ou non), sur membrane de seuil de coupure inférieur à 5 kDa, et supérieur à 1 kDa, afin d'obtenir un isolat protéique soluble, puis
- évaporation, pasteurisation et atomisation dudit isolat protéique.

### Choix de la microalgue

De préférence, les microalgues du genre Chlorella sont choisies dans le groupe constitué de *Chlorella vulgaris, Chlorella sorokiniana* et *Chlorella protothecoides,* et sont plus particulièrement *Chlorella protothecoides.*

Dans un mode de réalisation particulier, la souche est *Chlorella protothecoides* (souche UTEX 250 - *The Culture Collection of Algae at the University of Texas at Austin* - USA).

Dans un autre mode de réalisation particulier, la souche est *Chlorella sorokiniana* (souche UTEX 1663 - *The Culture Collection of Algae at the University of Texas at Austin* - USA).

La culture en conditions hétérotrophiques et en l'absence de lumière conduit classiquement à la production d'une biomasse de chlorelles présentant une teneur en protéines (évaluée par la mesure du taux d'azote N x 6,25) de 45 à 70 % en poids de cellules sèches.

Comme il sera exemplifié ci-après, cette culture est réalisée en deux étapes :
- préculture dans un milieu contenant du glucose et de l'extrait de levures pendant 72 h à 28 °C sous agitation, puis
- culture pour production de la biomasse proprement dite en glucose et extrait de levures pendant plus de 36 h à 28 °C, sous agitation et à pH 6,5 régulé à l'ammoniaque,
ce qui conduit à environ 80 g/L de biomasse à une teneur en protéines (évalué par le N x 6,25) de l'ordre de 52 % en poids de cellules sèches.

La biomasse est ensuite collectée par séparation solide-liquide, par filtration frontale ou tangentielle ou par tout moyen connu par ailleurs de l'homme du métier.

De manière optionnelle, la société Demanderesse recommande ensuite de laver la biomasse de manière à éliminer les composés solubles interstitiels par une succession de concentration (par centrifugation) / dilution de la biomasse.

Au sens de l'invention, on entend par « composés solubles interstitiels » tous les contaminants organiques solubles du milieu de fermentation, par exemple les composés hydrosolubles tels les sels, le glucose résiduel, les oligosaccharides de degré de polymérisation (ou DP) 2 ou 3 ou les peptides.

Cette biomasse ainsi purifiée de ses composés solubles interstitiels est ensuite ajustée préférentiellement à une matière sèche comprise entre 15 et 30 % en poids, de préférence à une matière sèche comprise entre 20 et 30 %.

### Perméabilisation thermique de la biomasse

On réalise alors le traitement thermique à une température comprise entre 50 et 150 °C, de préférence 100 et 150 °C, pendant une durée comprise entre 10 secondes et 5 minutes, de préférence pendant une durée comprise entre 10 secondes et 1 minute.

Ce traitement permet de laisser diffuser les composants intracellulaires dans le milieu réactionnel.

Enfin, au terme de ces étapes, on laisse refroidir à une température finale comprise entre 0° et 10 °C, de préférence à une température de l'ordre de 4 °C.

Sans être liée par une quelconque théorie, la société Demanderesse considère que le traitement thermique, réalisé dans ces conditions opératoires, pourrait agir ainsi comme un procédé de fragilisation membranaire qui autorise le relargage spontané des composants solubles du compartiment intracellulaire, voire de la matrice extracellulaire.

En plus des substances ioniques, des substances organiques tels que les hydrates de carbone (majoritairement DP1 et DP2), les peptides et les polypeptides sont drainés hors de la cellule.

Au contraire, les lipides et composés organiques hydrophobes restent dans les cellules, ce qui démontre bien que les cellules sont perméabilisées et non lysées ou détruites.

Le procédé selon l'invention ne conduit donc pas à la formation d'une émulsion, mais bien à une suspension aqueuse.

Le relargage de toutes ces substances solubles à travers la membrane perméabilisée s'apparente à un procédé de diffusion libre de type dialyse.

Par voie de conséquence, un temps de latence peut être nécessaire pour permettre une diffusion suffisante après le traitement thermique qui perméabilise la membrane.

Dans la littérature, le procédé de perméabilisation par champ pulsé des membranes de levures pour en extraire les protéines demande de 4 h à 6 h (Ganeva et al., 2003, Analytical Biochemistry, 315, 77-84).

Selon l'invention, un temps de réaction beaucoup plus court est mis en œuvre, compris entre 10 secondes et 5 minutes.

La biomasse résiduelle est ensuite éliminée par une technique de séparation solide-liquide par filtration frontale ou tangentielle, par floculation, par centrifugation, ou par tout moyen connu par ailleurs de l'homme du métier, ce qui permet de récupérer aisément la fraction soluble débarrassée des cellules de microalgues.

Au préalable de cette étape d'élimination de la biomasse, il est possible de procéder à une dilution des cellules perméabilisées afin d'améliorer le rendement et la qualité de cette étape de séparation solide-liquide.

La fraction soluble résultante est finalement composée essentiellement de protéines (50 - 80 % p/p) et d'hydrates de carbone (5 - 15 % p/p).

### Séparation membranaire

Les procédés classiques de récupération des protéines solubles reposent généralement sur une étape de précipitation desdites protéines par l'acide trichloracétique (10 % poids/volume) ou au sulfate d'ammonium.

Cependant, ces isolements par précipitation font suite à des procédés de cassage cellulaire très destructeurs (le plus souvent par sonication ou homogénéisation) qui, s'ils permettent en effet d'augmenter les rendements d'extraction, conduisent surtout à des protéines de faible solubilité et dénaturées.

Leur refonctionnalisation ne peut alors être envisagée que par le biais de leur produit d'hydrolyse (en peptides) par des moyens chimiques (lyse à la soude), physiques (traitement à haute température) ou enzymatiques (enzymes protéolytiques).

Le procédé selon l'invention permet bien au contraire de libérer des peptides et polypeptides natifs, intacts, dont toutes les fonctionnalités peuvent toujours s'exprimer.

Le procédé de l'invention conduit ensuite à l'isolement des protéines d'intérêt, par fractionnement membranaire.

La société Demanderesse recommande ainsi de procéder en deux étapes :
- éventuellement, récupération et clarification de la fraction soluble ainsi obtenue par microfiltration de manière à la débarrasser des insolubles résiduels,
- ultrafiltration de la fraction soluble (clarifiée ou non clarifiée, selon que l'étape précédente de récupération et clarification soit respectivement effectuée ou non), sur membrane de seuil de coupure inférieur à 5 kDa, et supérieur à 1 kDa, afin d'obtenir un isolat protéique soluble.

L'exploitation de ces voies permet de purifier les peptides et polypeptides solubles de leurs sels et sucres résiduels.

On obtient alors un isolat de protéines solubles ayant une teneur en protéines supérieure à 90 % en poids.

L'invention sera mieux comprise à l'aide des exemples qui suivent, lesquels se veulent illustratifs et non limitatifs.

### EXEMPLES

### Exemple 1 : Production de Chlorella protothecoides en fermentation de type fed-batch

La souche utilisée est *Chlorella protothecoides UTEX 250.*

### Pré-culture :

- 500 mL de milieu dans un Erlenmeyer de 2L ;
- Composition du milieu (en g/L) :

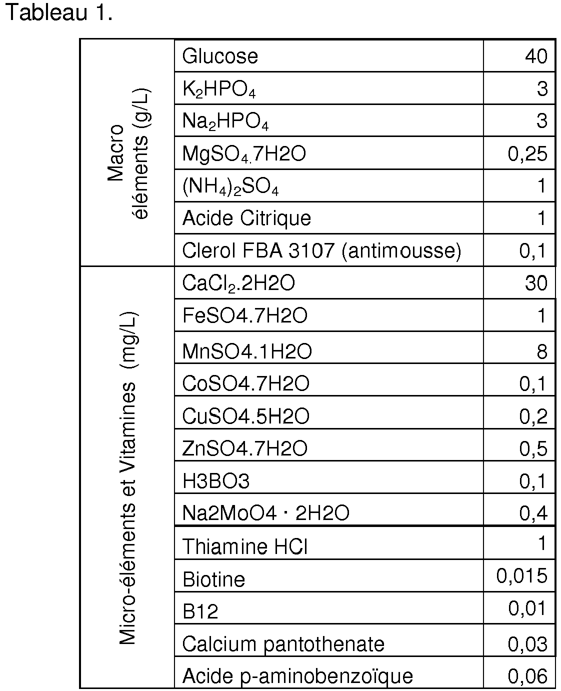

L'incubation se déroule dans les conditions suivantes : durée : 72 h ; température : 28 °C ; agitation : 110 rpm (Incubateur Infors Multitron).

La pré-culture est ensuite transférée dans un fermenteur de 30L de type Sartorius.

Culture pour production de biomasse :

Le milieu est le suivant :

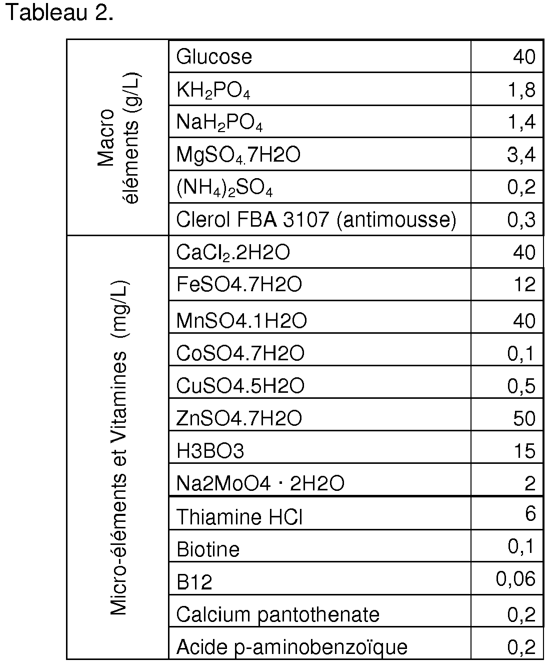

Le volume initial (Vi) du fermenteur est ajusté à 17 L après ensemencement. Il est porté à 20 à 25 L environ en final.

Les paramètres de conduite de la fermentation sont les suivants :

**Tableau 3.**

| | |
|---|---|
| Température | 28 °C |
| pH | 5,0 - 5,2 par NH₃ 28% w/w |
| pO₂ | 20% +/- 5% (maintenue par agitation) |
| Agitation | 300 RPM mini |
| Débit d'air | 15 L/min |

Lorsque la concentration résiduelle en glucose tombe en dessous de 10 g/L, un apport de glucose sous forme d'une solution concentrée à 800 g/L environ est réalisé de façon à maintenir la teneur en glucose entre 0 et 20 g/L dans le fermenteur.

### Résultats

En 40h, on obtient 80 g/L de biomasse contenant 52 % de protéines.

### Exemple 2. Perméabilisation thermique de la biomasse de Chlorella protothecoides et récupération de la fraction soluble

La biomasse obtenue selon l'exemple 1 est :
- centrifugée et lavée de manière à être amenée à une matière sèche de 220 g/l et à une pureté de plus 90 % (pureté définie par le rapport entre la matière sèche de la biomasse sur la matière sèche totale), puis
- traitée thermiquement par UHT sur un barème d'une dizaine de secondes à 135 °C.

La biomasse « partiellement solubilisée » ainsi obtenue présente de l'ordre de 50 % de peptides et protéines (exprimés en acides aminés totaux), 20 % de sucres et 15 % de lipides, correspondant à un taux de solubilisation entre 20 et 50% relativement à la biomasse totale initiale.

Elle est ensuite séparée de la fraction soluble par séparation centrifuge.

Les solubles « bruts » renferment alors entre 60 et 75% de peptides et protéines (exprimés en acides aminés totaux - dont 90 % d'arginine et acide glutamique), entre 10 et 25 % de sucres et moins de 1% de lipides.

La biomasse « appauvrie », dans le culot de centrifugation, présente quant à elle encore entre 20 et 35 % de peptides et protéines (exprimés en acides aminés totaux), 25 à 35 % de sucres et surtout entre 20 à 25 % de lipides.

Le perméat « P1 » de microfiltration présentant une matière sèche de 5 % et titrant entre 60 et 75 % de peptides et protéines (exprimés en acides aminés totaux) est ensuite ultrafiltré sur membrane de seuil de coupure < 5 kDa.

Le rétentat « R2 » d'ultrafiltration ainsi obtenu présente 10 % (5 à 20%) de matière sèche, et contient plus de 90 % de peptides présentant un poids moléculaire supérieur ou égal à 5 kDa.

Le perméat « P2 » inférieur à 3 % de matière sèche, contient quant à lui des peptides présentant un poids moléculaire inférieur à 5 kDa et des oligosaccharides de DP inférieur ou égal à 2.

Ce perméat « P2 » peut alors être notamment filtré sur membrane d'osmose inverse (présentant un taux de réjection en NaCl de 93 %), de manière à obtenir :
∘ un rétentat « R3 » à plus de 10% de matière sèche contenant des peptides présentant un poids moléculaire inférieur à 5 kDa et des oligosaccharides de DP 2, tel le saccharose ;
∘ un perméat « R3 » à 0,1 % de matière sèche contenant des oligosaccharides de DP1, des sels, des acides aminés libres et des acides organiques.
L'isolat protéique « R2 » est alors :
- neutralisé à pH 7 à la potasse,
- concentré par évaporation à 35% de matière sèche (MS),
- pasteurisé et
- atomisé.

## Revendications

1. Procédé de préparation d'un isolat protéique de la biomasse de microalgues du genre *Chlorella,* **caractérisé en ce qu'**il comprend les étapes suivantes :
- fourniture d'une biomasse de microalgues produite par fermentation,
- de manière optionnelle, lavage de la biomasse de manière à éliminer les composés solubles interstitiels,
- perméabilisation thermique de la biomasse à une température comprise entre 50 et 150°C, de préférence 100 et 150°C, pendant une durée comprise entre 10 secondes et 5 minutes, de préférence pendant une durée comprise entre 10 secondes et 1 minute,
- élimination de la biomasse ainsi perméabilisée par une technique de séparation solide-liquide choisie dans le groupe constitué de la filtration frontale ou tangentielle, la floculation et la centrifugation, plus particulièrement la centrifugation multi-étagée, pour obtenir une fraction soluble,
- de manière optionnelle, récupération et clarification de la fraction soluble ainsi obtenue par microfiltration de manière à la débarrasser des insolubles résiduels,
- ultrafiltration de la fraction soluble, sur membrane de seuil de coupure inférieur à 5 kDa et supérieur à 1 kDa, afin d'obtenir un isolat protéique soluble, puis
- évaporation, pasteurisation et atomisation dudit isolat protéique.

2. Procédé selon la revendication 1, **caractérisé en ce que** les microalgues du genre *Chlorella* sont choisies dans le groupe constitué de *Chlorella vulgaris, Chlorella sorokiniana* et *Chlorella protothecoides,* et sont plus particulièrement *Chlorella protothecoides.*

## Patentansprüche

1. Verfahren zur Herstellung eines Proteinisolats aus der Biomasse von Mikroalgen der Gattung *Chlorella,* **dadurch gekennzeichnet, dass** es die folgenden Schritte umfasst:
- Bereitstellen einer mittels Fermentation produzierten Mikroalgenbiomasse,
- optional Waschen der Biomasse, um interstitielle lösliche Verbindungen zu entfernen,
- thermische Permeabilisierung der Biomasse bei einer Temperatur zwischen 50 und 150°C, vorzugsweise zwischen 100 und 150°C in einem Zeitraum zwischen 10 Sekunden und 5 Minuten, vorzugsweise in einem Zeitraum zwischen 10 Sekunden und 1 Minute,
- Entfernen der auf diese Weise permeabilisierten Biomasse mithilfe eines fest-flüssig Trennverfahrens, ausgewählt aus der Gruppe, bestehend aus Frontal- oder Tangentialfiltration, Koagulation und Zentrifugation, insbesondere mehrstufiger Zentrifugation, um eine lösliche Fraktion zu erhalten,
- optional Wiedergewinnung und Klärung der auf diese Weise erhaltenen löslichen Fraktion mithilfe Mikrofiltration, um unlösliche Rückstände zu entfernen,
- Ultrafiltration der löslichen Fraktion auf einer Membran mit einer Ausschlussgrenze unter 5 kDa und über 1 kDa, um ein lösliches Proteinisolat zu erhalten, anschließend
- Eindampfen, Pasteurisieren und Zerstäuben besagten Proteinisolats.

2. Verfahren gemäß Anspruch 1, **dadurch gekennzeichnet, dass** die Mikroalgen der Gattung *Chlorella* aus der Gruppe ausgewählt sind, bestehend aus *Chlorella vulgaris, Chlorella sorokiniana* und *Chlorella protothecoides,* und insbesondere *Chlorella protothecoides* sind.

## Claims

1. A method for preparing a protein isolate from the biomass of microalgae of the *Chlorella* genus, **characterized in that** it comprises the following steps:
- providing a microalgal biomass produced by fermentation,
- optionally, washing the biomass so as to eliminate the interstitial soluble compounds,
- thermal permeabilization of the biomass at a temperature of between 50 and 150°C, preferably 100 and 150°C, for a duration of between 10 seconds and 5 minutes, preferably for a duration of between 10 seconds and 1 minute,
- elimination of the biomass permeabilized in this way by a technique of solid-liquid separation chosen from the group consisting of frontal or tangential filtration, flocculation and centrifugation, more particularly multistage centrifugation, to obtain a soluble fraction,
- optionally, recovery and clarification of the soluble fraction obtained in this way by microfiltration so as to remove residual insoluble substances therefrom,
- ultrafiltration of the soluble fraction on a membrane with a cut-off threshold of less than 5 kDa and more than 1 kDa, so as to obtain a soluble protein isolate, then
- evaporation, pasteurization and atomization of said protein isolate.

2. The method as claimed in claim 1, **characterized in that** the microalgae of the *Chlorella* genus are chosen from the group consisting of *Chlorella vulgaris, Chlorella sorokiniana* and *Chlorella protothecoides,* and are more particularly *Chlorella protothecoides.*
